# EUROPÄISCHE PATENTSCHRIFT

(11) **EP 0 606 639 B1**
(45) Veröffentlichungstag und Bekanntmachung des Hinweises auf die Patenterteilung: **10.04.2002**
(21) Anmeldenummer: 93120882.1
(22) Anmeldetag: 24.12.1993
(51) Int. Cl.: G01N 21/90

(54) **Verfahren und Vorrichtung zum Prüfen von Flaschen**
Method and device for inspecting bottles
Procédé et dispositif d'inspection de bouteilles

(30) Priorität: 07.01.1993 DE 4300169
(43) Veröffentlichungstag der Anmeldung: 20.07.1994
(73) Patentinhaber: UNISENSOR Sensorsysteme GmbH, 76149 Karlsruhe (DE)
(72) Erfinder: Schrader, Bernard, Prof.Dr.-Ing., 45259 Essen (DE); Hoffmann, Günter Georg, Dr., 46045 Oberhausen (DE)
(74) Vertreter: Neidl-Stippler, Cornelia, Dr.

(56) Entgegenhaltungen:
- WO-A-88/00862
- WO-A-89/09390
- US-A- 3 394 263
- US-A- 4 778 999
- APPLIED SPECTROSCOPY, Bd.46, Nr.4, 1992 Seiten 568 - 570, XP264016 G. G. HOFFMANN ET AL. 'Combined Raman and Fluorescence Spectroscopy with the Same Compact CCD-Based Instrument'

## Beschreibung

Die Erfindung betrifft ein Verfahren zum optischen Testen transparenter Behälter insbesondere von farblosen Kunststoffflaschen nach Teil 1 des Anspruches 1. Dabei wird eine optische Prüfstrahlung auf eine zu prüfende Fläche eines Behälters ausgerichtet, die von der beleuchteten Behälterfläche ausgehende Strahlung in Folge des Einfalls der Prüfstrahlung erfaßt und in Abhängigkeit von der erfaßten Strahlung bestimmten Qualitätsmerkmalen des Behälters entsprechende Prüfsignale gebildet.

Die Erfindung betrifft weiter eine Vorrichtung zum optischen Prüfen transparenter Behälter, insbesondere von farblosen Kunststoffflaschen, mit einem die zu prüfenden Behälter nach einander in eine Prüfzone fördernden Behälterförderer, einer Prüfeinrichtung, welche eine Strahlungsquelle zum Aussenden einer optischen Prüfstrahlung, Strahlführungsmittel zum Führen und Ausrichten der Prüfstrahlung auf einen zu prüfenden Behälter in der Prüfzone und zum Empfangen und Führen einer aufgrund des Einfalls der Prüfstrahlung von der Prüfzone ausgehenden Meßstrahlung und Sensormittel zum Erfassen und Verarbeiten der Meßstrahlung zu entsprechenden Meßsignalen aufweist, und einer Auswertanordnung zum Verarbeiten der Meßsignale zu wenigstens einem Qualitätsmerkmal des betreffenden Behälters entsprechenden Prüfsignale.

Im Getränkehandel werden zunehmend Mehrwegflaschen aus Kunststoff, insbesondere aus PET (Polyethylenterephthalat) eingesetzt.
Diese Kunststoffe, und insbesondere auch das PET, bilden zwar eine glatte Oberfläche, sind aber von der Oberfläche her quellfähig. Das bedeutet, daß Chemikalien, die chemisch ähnlich aufgebaut sind wie PET, in die oberflächennahen Strukturen des PET eindringen können und sich zwischen die langkettigen Moleküle einlagern. Im täglichen Gebrauch kann das zu nicht unerheblichen Problemen führen, wenn die Getränkeflaschen zwischendurch zum Aufbewahren beliebiger Flüssigkeiten zweckentfremdet werden. Werden z.B. Flüssigkeiten wie Altöl, Benzin oder ein Herbizid in eine PET-Mehrwegflasche gefüllt, so können diese Stoffe oder Bestandteile dieser Stoffe in die intermolekularen Räume in der Kunststoffwand der Flasche eindiffundieren und darin festgehalten werden. Wird eine solche Flasche später gereinigt und wieder mit einem Getränk gefüllt, so können die in die Flaschenwand eingedrungenen Stoffe herausdiffundieren und das in die Flasche gefüllte Getränk ungenießbar oder gar gesundheitsschädlich machen. Auch Aromastoffe von Fruchtsäften oder anderen aromatisierten Getränken können in die Wandung der Flasche eintreten und so bei späterer Wiederbefüllung mit einem anderen Getränk zu einer Geschmacksbeeinträchtigung führen. Besonders kritisch ist dies für das Abfüllen von Mineralwasser, wo sich Geschmacksbeeinflussungen durch Spuren früher in der Flasche enthaltener aromatisierter Getränke sehr nachteilig auswirken können.

Derart durch Eindiffundieren von Substanzen in die Innenwandfläche der Flaschen kontaminierte Flaschen können durch die heute üblichen und bekannten Renigungssysteme nicht wieder brauchbar gemacht werden. Derartige Flaschen müssen zuverlässig ausgesondert werden. Es gibt daher in der Getränkeabfüllindustrie Inspektionsgeräte, welche Gefahrstoffe und Verunreinigungen entdecken und kontaminierte Flaschen ausschleusen sollen.

Es ist bekannt, transparente Flaschen zum Zwecke des Prüfens quer durch die Wand oder in Achsrichtung durch die Öffnung und den Flaschenboden hindurch zu durchleuchten und die Intensität des durch die Flasche hindurchtretenden Lichts zu erfassen, um daraus Erkenntnisse über den Zustand der Flasche zu gewinnen. Das erlaubt es, Beschädigungen der Flasche und größere Fremdkörper oder Verunreinigungen zu erkennen, aber schon das Auffinden kleinerer Fehler ist nicht mehr zuverlässig gewährleistet. Substanzen, die nur in Spuren an der Flaschenwand haften oder in sie hineindiffundiert sind, lassen sich mit den bekannten Meßmethoden nicht mehr feststellen. Das trifft auch auf moderne Schnüffelverfahren zu, die Aerosole freisetzende Substanzen in den Flaschen aufsparen sollen. Sie sind für moderne Fertigungsanlagen zu langsam und zu ungenau, um auch noch sehr kleine Spuren kritischer Substanzen zu entdecken.

Aus der WO 88/00862 ist ein Verfahren zur Analyse von Rückständen in Behältern bekannt geworden. Bei diesem werden Rückstände aus den Behältern, die gegebenenfalls in einem Lösungsmittel gelöst wurden, in eine Küvette überführt und von dieser Lösung der Rückstände in an sich bekannter Weise Spektren ermittelt. Es werden also die Reststoffe aus den Behältern herausgewaschen und dann in einer getrennten Anordnung untersucht. Demgegenüber ist Aufgabe der Erfindung, auch sehr kleine Mengen kritischer Substanzen bereits im Inneren von Mehrwegbehältern sicher zu erkennen und dann die kontaminierten Behälter aussondern zu können.

Die Aufgabe wird erfindungsgemäß durch ein Verfahren mit den Merkmalen des Patentanspruchs 1 gelöst. Weiterhin bezieht sich die Erfindung auf eine Vorrichtung gemäß Patentanspruch 10. Vorteilhafte Weiterbildungen ergeben sich aus den abhängigen Ansprüchen.

Bei einem Verfahren der eingangs angegebenen Art wird die Aufgabe also erfindungsgemäß dadurch gelöst, daß mit der Prüfstrahlung im Bereich der Behälterinnenwand eine Streustrahlung erzeugt wird und daß diese Streustrahlung erfaßt und zur Bildung der Prüfsignale analysiert und ausgewertet wird. Die durch die einfallende Prüfstrahlung erzeugte Streustrahlung hängt von der Beschaffenheit der inneren Wandfläche der Behalter und ggf. von vorhandenen fein verteilten Verunreinigungen ab, die somit durch Erfassen der aus dem Behälterinnern heraustretenden Streustrahlung erkannt werden können. Die in Abhängigkeit von der Streustrahlung gebildeten Prüfsignale klassifizieren die Behälter als ordnungsgemäß oder fehlerhaft bzw. kontaminiert und bewirken das Ausschleusen der nicht akzeptablen Behalter.

Eine bevorzugte Weiterbildung des Verfahrens nach der Erfindung besteht darin, daß die Prüfstrahlung auf einen Abschnitt der Behälterinnenwand fokussiert wird und daß eine in diesem Abschnitt durch den Einfall der Prüfstrahlung erzeugte Streustrahlung erfaßt und zur Bildung von wenigstens eine charakteristische Eigenschaft der Innenwandfläche repräsentierenden Prüfsignalen analysiert und verarbeitet wird. Durch das Fokussieren der Prüfstrahlung auf die Behälterinnenwand wird in diesem Bereich eine intensive Streustrahlung erzeugt, sofern der Zustand der Behälterinnenwand und/oder ihre Kontaminierung mit unerwünschten Substanzen die Bedingungen dafür bieten. Eine weitere bevorzugte Fortbildung des Verfahrens nach der Erfindung besteht darin, daß als Prüfstrahlung eine monochromatische Strahlung auf die Behälterinnenwand fokussiert wird, daß eine gegenüber der einfallenden Prüfstrahlung in Abhängigkeit von wenigstens einer im Bereich der Behälterinnenwand vorhandenen Substanz veränderte Streustrahlung (Raman-Strahlung) erfaßt wird, daß diese Streustrahlung spektral analysiert wird und daß in Abhängigkeit von der Struktur des Spektrums der Streustrahlung charakteristische Qualitätsmerkmale und/oder Bestandteile im Bereich der Behälterinnenwand repräsentierende Prüfsignale gebildet werden. Das Fokussieren der monochromatischen Strahlung auf die Behälterinnenwand erzeugt beim Vorhandensein entsprechender Substanzen eine für eine Spektralanalyse ausreichend intensive Raman-Streustrahlung. Die Spektralanalyse dieser Raman-Strahlung gibt zuverlässig Auskunft über am Ort der Streustrahlung vorhandene Substanzen.

In weiterer Fortführung der Erfindung ist vorgesehen, daß durch die einfallende Prüfstrahlung wenigstens eine im Bereich der Behälterinnenwand vorhandene Substanz zur Abgabe einer Fluoreszenzstrahlung angeregt wird, daß die Fluoreszenzstrahlung erfaßt und spektral zerlegt wird und daß in Abhängigkeit von der Struktur des Fluoreszenzspektrums charakteristische Qualitätsmerkmale und/oder Bestandteile im Bereich der Behälterinnenwand repräsentierende Prüfsignale gebildet werden. Die Streu- und/oder Fluoreszenzstrahlung kann zur Bildung eines Beugungsspektrums an einem Beugungsgitter spektral zerlegt werden. Die erfindungsgemäß vorgesehene Erfassung und spektrale Analyse der Fluoreszenzstrahlung gibt Aufschluß über Substanzen im Bereich der Behälterinnenwand, die mit der Raman-Strahlung nicht oder nur unzureichend erfaßt werden können. Die Spektralanalyse der Fluoreszenzstrahlung vermittelt Informationen über das Vorhandensein von Spuren von Maschinenöl, Benzin oder bestimmten Pestiziden und Herbiziden.

Besonders vorteilhaft gestaltet sich das Verfahren nach der Erfindung dadurch, daß charakteristische Daten wenigstens eines Streulicht- und/oder Fluoreszenzlichtspektrums gespeichert werden, daß entsprechende charakteristische Daten jedes beim Prüfen eines Behälters aktuell erzeugten Spektrums mit den gespeicherten Daten verglichen und entsprechende Vergleichssignale gebildet werden und daß die geprüften Behälter in Abhängigkeit von den Vergleichsignalen als ordnungsgemäß oder fehlerhaft klassifiziert werden. Dadurch ist es möglich, ein Spektrum oder mehrere Spektren ordnungsgemäßer Behälter in der Auswertanordnung zu speichern und laufend mit den in aktuellen Prüfvorgängen gewonnenen Spektren zu vergleichen. Weichen die aufgrund der Prüfvorgänge erhaltenen Spektren von den gespeicherten Spektren ab, so werden die betreffenden Behälter als fehlerhaft klassifiziert. Die Erfindung sieht auch vor, daß die charakteristischen Daten der Streulicht- und/oder Fluoreszenzlichtspektren ausgewählter Substanzen gespeichert werden, daß entsprechende charakteristische Daten jedes beim Prüfen eines Behälters aktuell erzeugten Spektrums mit den gespeicherten Daten der ausgewählten Substanzen verglichen und entsprechende Vergleichssignale gebildet werden und daß die geprüften Behälter in Abhängigkeit von den Vergleichssignalen klassifiziert werden. Dieses Vorgehen bietet eine Möglichkeit, die Behälter aufgrund der optischen Prüfung entsprechend ihren Verunreinigungen zu klassifizieren. Damit können die unakzeptablen Behälter nach Fehlerart sortiert aus dem aktuellen Fertigungs- oder Bearbeitungsprozeß ausgeschleust und gezielt weiteren bzw. anderen Bearbeitungsprozessen zugeführt werden.

Gemäß einer Weiterbildung der Erfindung ist vorgesehen, daß eine in Phase und/oder Wellenlänge gegenüber der einfallenden Prüfstrahlung unveränderte zweite Streustrahlung (Rayleigh-Streuung) erfaßt wird und daß in Abhängigkeit von der Intensität dieser zweiten Streustrahlung vorgegebene charakteristische Eigenschaften und/oder Bestandteile der Behälterinnenwand repräsentierende zweite Prüfsignale gebildet werden. Das erfindungsgemäß vorgeschlagene Erfassen der Rayleigh-Streuung führt zum Erkennen trüber Beläge auf der Innenseite der Behälter und erhöht somit die Zuverlässigkeit des Verfahrens nach der Erfindung.

Eine weitere Verfahrensausgestaltung sieht vor, daß die zu prüfenden Behälter nacheinander in den Fokusbereich der optischen Prüfstrahlung gefördert werden, daß jeder Behälter während seines Aufenthalts im Fokusbereichs der Prüfstrahlung zur Bildung von wenigstens zwei Prüfsignalen wenigstens zweimal geprüft wird, daß die Position des Behälters zur einfallenden Prüfstrahlung zwischen aufeinanderfolgenden Prüfvorgängen verändert wird und daß aus den einem Behälter zugeordneten Prüfsignalen ein Mittelwertsignal erzeugt wird. Als Prüfstrahlung wird vorzugsweise eine Laserstrahlung im optischen Wellenlängenbereich, insbesondere im ultravioletten, sichtbaren oder nahen infraroten Bereich des elektromagnetischen Spektrums verwendet.

Bei einer Vorrichtung der eingangs angegebenen Art wird die der Erfindung zugrunde liegende Aufgabe erfindungsgemäß dadurch gelöst, daß die Strahlungsquelle zum Erzeugen einer im Bereich des Behälterinnern eine Streustrahlung hervorrufenden Prüfstrahlung ausgebildet ist und daß die Prüfeinrichtung Mittel zum Erfassen und Analysieren dieser Streustrahlung und zur Bildung entsprechender Prüfsignale aufweist.

Weitere Fortführungen und Ausgestaltungen der Vorrichtung nach der Erfindung sind in den Unteransprüchen 12 bis 18 enthalten. Dabei enthalten die Ansprüche 12 bis 14 bevorzugte Ausbildungen der Strahlungsquelle und der Strahlführungsmittel der Prüfeinrichtung. Die Ansprüche 15 und 16 beziehen sich auf die bevorzugte Ausbildung der Sensormittel als Spektrometer zur spektralen Analyse der empfangenen Strahlung. Die Ansprüche 17 und 18 enthalten Merkmale vorteilhafter und bevorzugter Ausbildungen der Auswertanordnung, welche für die Auswertung der durch die Spektralanalyse gewonnenen Spektren vorgesehen ist.

Die Erfindung bietet den Vorteil, daß aufgrund der Fokussierung der Prüfstrahlung auf die Behälterinnenwand und der spektralen Analyse der dabei erzeugten Streustrahlung auch kleine Mengen kritischer Substanzen erkannt werden können, selbst, wenn sie in die Behälterinnenwand hineindiffundiert sind. Auch das Behältermaterial selbst kann mit diesen Messungen unterschieden werden, so daß aus verschiedenen Kunststoffen bzw. aus Glas hergestellte Behälter erkannt werden. So können beispielsweise Behälter aus PET (Polyäthylenterephthalat), PVC (Polyvinylchlorid), PE (Polyäthylen) oder PP (Polypropylen) oder aus Glas voneinander unterschieden und entsprechend klassifiziert werden. Da die Methode nach der Erfindung auch kleine Mengen kritischer Substanzen erfaßt, bietet sie eine zuverlässige Möglichkeit, fehlerhafte bzw. auch geringfügig kontaminierte Behälter auszusondern. Da in der Auswertanordnung Spektren ausgewählter Substanzen vorgegeben werden können, können nicht akzeptable Behälter nach Qualitätskriterien sortiert, ausgeschleust und weiterbehandelt werden. Das Verfahren und die Vorrichtung nach der Erfindung bieten kurze Prüfzeiten, so daß die vorgeschlagene Prüfung auch in moderne Hochleistungsmaschinen integriert werden kann. Dabei ist der konstruktive Aufwand für die Vorrichtung relativ gering.

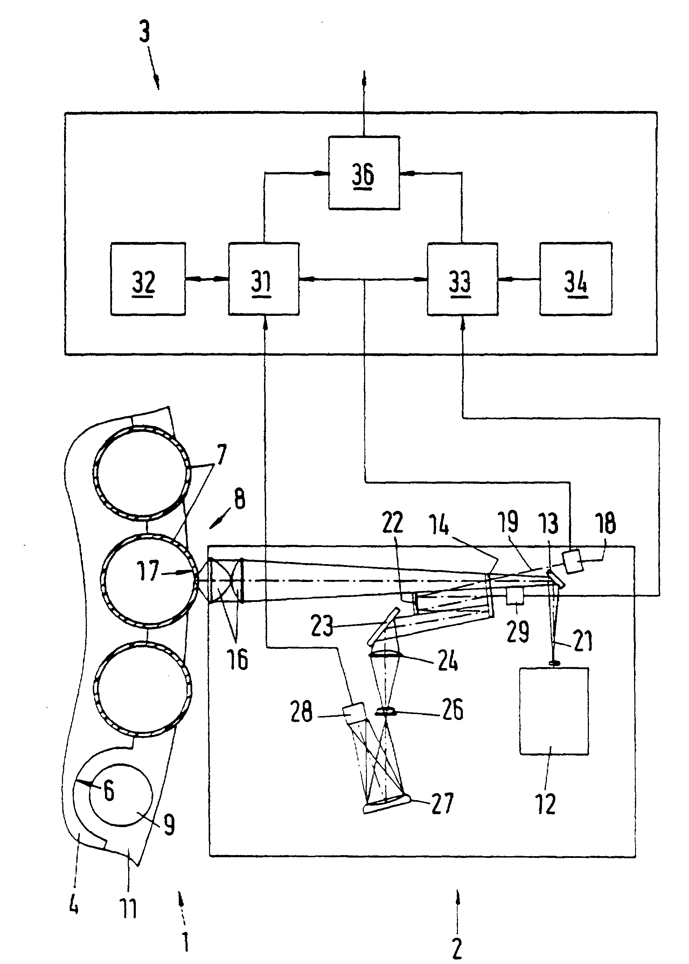

Die Erfindung wird nun anhand der Zeichnung näher erläutert.

Die einzige Figur zeigt in schematischer Darstellung eine optische Prüfvorrichtung nach der Erfindung.

Die in der Zeichnung dargestellte Prüfvorrichtung zeigt einen Behälterförderer 1, eine Prüfeinrichtung 2 und eine mit der Prüfeinrichtung verbundene Auswertanordnung 3.

Der Behälterförderer 1 ist als schrittweise antreibbarer Förderstern 4 ausgebildet, der an seinem Umfang Aufnahmen 6 zum Aufnehmen, Transportieren und Positionieren von zu prüfenden Behältern 7 aufweist. Bei den Behältern 7 handelt es sich insbesondere um Mehrweg-PET-Flaschen, die einen Reinigungsprozeß durchlaufen haben und in weiteren folgenden Schritten erneut gefüllt, verschlossen, etikettiert usw. werden sollen. Da die vorangegangene Behandlung und Verwendung der Flaschen durch die Konsumenten der Getränke nicht bekannt ist, ist vor der Weiterverwendung der Flaschen eine intensive Prüfung auf Unbedenklichkeit und Fehlerfreiheit erforderlich. Dabei muß jede einzelne Flasche sorgfältig geprüft werden, weil die vom Markt zurückgeführten Flaschen ganz unterschiedliche Behandlung erfahren haben, so daß gelegentliche Stichproben nicht ausreichen. Der Behälterförderer 1 ist daher so angetrieben, daß jeder Behälter 7 sich lange genug in einer Prüfzone 8 befindet, um die notwendigen optischen Prüfungen vornehmen zu können.

Die Behälter 7 sind in ihren Aufnahmen 6 auf Drehtellern 9 abgestellt, die auf einer zusammen mit dem Förderstern 4 bewegten Stützscheibe oder einem Tragring 11 drehbar angeordnet sind. Der Prüfzone 8 ist ein nicht dargestellter Antriebsmechanismus zugeordnet, der den Drehteller 9 und den darauf befindlichen Behälter 7 wenigstens einmal um einen vorgegebenen Winkel verdreht, während dieser sich in der Prüfzone befindet, sofern eine mehrmalige Prüfung ein und desselben Behälters für erforderlich gehalten wird.

Die Prüfeinrichtung 2 weist als monochromatische Strahlungsquelle 12 einen Laser, beispielsweise einen mit Neodym dotierten Yttrium-Aluminium-Granat-Laser (einen sogenannten Nd: YAG-Laser) mit Frequenz-Verdopplung bzw. -Verdreifachung, auf, der eine monochromatische Strahlung 21 im ultravioletten Wellenlängenbereich des elektromagnetischen Spektrums abgibt. Es können auch andere Laser oder monochromatische Strahlungsquellen verwendet werden, deren Strahlung im optischen Wellenlängenbereich, also im ultravioletten, sichtbaren oder nahen infraroten Bereich des Spektrums liegt. Die von dem Laser 12 abgegebene Strahlung wird von einem Spiegel 13 umgelenkt, tritt durch ein Interferenzfilter 14 hindurch und wird von einer Linsenoptik 16 in den Bereich der Behälterinnenwand 17 fokussiert. Ein Abschnitt der Behälterinnenwand 17 eines in der Prüfzone 8 positionierten Behälters 7 liegt also etwa im Brennpunkt der Linsenoptik 16, so daß nahezu die gesamte von dem Laser 12 ausgehende Strahlung 21 in diesem Abschnitt der Behälterinnenwand konzentriert wird. Nur ein geringer Bruchteil 19 der Laserstrahlung 21 wird als Referenzstrahlung am Interferenzfilter 14 reflektiert und gelangt zu einem Referenzstrahlsensor 18, welcher ein dieser Referenzstrahlung 19 entsprechendes Referenzsignal an die Auswertanordnung 3 abgibt.

Die Prüfstrahlung 21 erzeugt im Bereich der Behälterinnenwand 17 eine Streustrahlung, die von der Linsenoptik 16 aufgefangen, und zweimal vom Interferenzfilter 14 reflektriert wird. Das Interferenzfilter 14 läßt die monochromatische Erregerstrahlung weitgehend durch und reflektiert Strahlung anderer Wellenlängen. Dadurch bewirkt es einerseits eine spektrale Reinigung der Laserstrahlung 21, indem es auf dem Weg zum Behälter den Laserstrahl begleitendes Licht (z.B. Licht des Anregungsplasmas) zurückhält. Andrerseits reinigt das Interferenzfilter die von dem Behälter zurückgesandte relativ schwache Streustrahlung von der starken Laserstrahlung, die von dem Filter durchgelassen wird, während die Streustrahlung überwiegend reflektiert wird. Die Streustrahlung wird wenigstens zweimal auf dem Interferenzfilter 14 reflektiert, um dessen Filterwirkung zu erhöhen. Zusätzlich oder anstelle des Filters 14 können auch andere Filter (nicht dargestellt) in den Strahlengang eingesetzt werden, z.B. eines, das die die Streustrahlung begleitende Laserstrahlung schwächt und eine hohe Transmission für Strahlung anderer Wellenlängen, also die Streustrahlung, aufweist. Das vom Interferenzfilter 14 reflektierte Licht wird über zwei weitere Spiegel 22 und 23, eine Sammellinse 24 und durch einen Spalt 26 zu einem Beugungsgitter 27 gelenkt. Als Beugungsgitter 27 ist vorzugsweise ein durch einen holographischen Prozess hergestelltes Konkavgitter vorgesehen. Die empfangene Streustrahlung wird an dem Beugungsgitter 27 spektral zerlegt und auf einem CCD-Sensor 28 als Spektrum abgebildet. Der Sensor 28 tastet das Spektrum der Streustrahlung ab und übermittelt die charakteristischen Daten des Spektrums an die Auswertanordnung 3.

Enthält die von der Linsenoptik 16 aus dem Fokusbereich an der Behälterinnenwand 17 empfangene Strahlung Anteile einer diffus reflektierten Strahlung, so wird ein Teil dieser Strahlung (Rayleigh-Strahlung) durch das Interferenzfilter 14 ausgekoppelt und von einem Sensor 29 erfaßt, der ein der Intensität dieser Strahlung entsprechendes Meßsignal an die Auswertanordnung 3 abgibt.

Die Auswertanordnung 3 weist einen ersten Prozessor 31 zum Verarbeiten der von dem Sensor 28 abgegebenen Daten der erfaßten Spektren, einen Datenspeicher 32 zum Speichern charakteristischer Daten der Spektren von ausgewählten Substanzen, einen zweiten Prozessor 33 zum Verarbeiten der von dem Sensor 29 abgegebenen Meßsignale, einen Grenzwertspeicher 34 und eine Steuereinheit 36 zum Verarbeiten der von den Prozessoren 31 und 33 abgegebenen Signale zu Steuersignalen bzw. Klassifizierungssignalen auf.

Zum Prüfen eines Behälters 7 wird dieser in der Prüfzone 8 positioniert und für die Dauer der optischen Prüfung angehalten. Die von dem Laser 12 abgegebene Strahlung 21 wird in einen Abschnitt der Behälterinnenwand 17 fokussiert, wodurch dort eine Streustrahlung erzeugt wird, die sich je nach den Eigenschaften der Innenwandfläche und nach den im Bereich der Innenwandfläche enthaltenen Substanzen aus verschiedenen Komponenten zusammensetzt. Trübe Flüssigkeiten können auf der Innenwand 17 des Behälters einen Belag bilden, der die Prüfstrahlung 21 diffus streut (Rayleigh-Strahlung). Auch eine durch agressive Mittel aufgerauhte innere Oberfläche kann diese diffuse Streustrahlung hervorrufen. Ein Teil dieser Rayleigh-Strahlung gelangt durch die Linsenoptik 16 in den Strahlengang der Prüfvorrichtung, wird an dem Interferenzfilter 14 und dem Spiegel 22 reflektiert und zum Teil durch das Interferenzfilter 14 hindurch zum Sensor 29 hin ausgekoppelt. Der Sensor 29 erzeugt ein der Intensität dieser diffusen Streustrahlung entsprechendes Meßsignal, das zum zweiten Prozessor 33 hin abgegeben wird, der es mit dem im Grenzwertspeicher 34 festgelegten Grenzwert vergleicht. Überschreitet dieses Meßsignal den Grenzwert, so erzeugt der Prozessor 33 ein Vergleichssignal, welches die Steuereinheit 36 zu einem Steuersignal verarbeitet, mit dem der betreffende Behälter 7 aus der Reihe der ordnungsgemäßen Behälter ausgeschleust wird.

Eine zweite Komponente der im Bereich der Behälterinnenwand 17 erzeugten Streustrahlung wird von einer gegenüber der einfallenden Prüfstrahlung 21 in Abhängigkeit von wenigstens einer im Bereich der Behälterinnenwand vorhandenen Substanz veränderten Streustrahlung (Raman-Strahlung) gebildet. Diese Raman-Strahlung wird von der Linsenoptik 16 erfaßt, von dem Filter 14 bzw. den Spiegeln 22 und 23 umgelenkt und von der Linse 24 zum Spalt 26 geführt, durch den hindurch sie zu dem Konkavgitter 27 gelangt. Das Konkavgitter 27, gemäß der Erfindung vorzugsweise ein holographisch hergestelltes Gitter, erzeugt auf dem CCD-Sensor 28 ein zum langwelligen Bereich des elektromagnetischen Spektrums hin verschobenes Linienspektrum, dessen charakteristische Daten auf das Behältermaterial und auf Fremdstoffe und kritische Substanzen an der Innenseite und im Wandmaterial des Behälters schließen lassen. Die charakteristischen Daten der Linien dieses Spektrums werden zum Prozessor 31 der Auswertanordnung 3 weitergegeben. Im Speicher 32, der mit dem Prozessor 31 verbunden ist, sind die charakteristischen Daten der Linien von Spektren ausgewählter Substanzen abgespeichert. Hier können die charakteristischen Daten der Spektrallinien von Substanzen wie Haushaltslösungsmitteln, Pinselreinigern, Aceton, Alkoholen, Verdünnern, Pflanzenschutzmitteln usw. abgelegt sein. Auch die Daten von Linienspektren von gängigen Flaschenmaterialien wie PET, Polyäthylen, PVC, Polypropylen, Glas usw. können hier gespeichert sein. Auch die Daten der Spektrallinien einer ordnungsgemäßen, fehlerfreien Behälterinnenwand sind hier vorteilhafter Weise zu speichern. Der Prozessor 31 vergleicht die Daten der charakteristischen Linien des jeweils bei einer Prüfung aktuell erzeugten Linienspektrums mit den Daten der gespeicherten Spektren der ausgewählten Substanzen und bildet Vergleichssignale, welche die geprüften Behälter 7 entsprechend klassifizieren. Diese Vergleichssignale gelangen zur Steuereinheit 36, welche in Abhängigkeit von den Vergleichssignalen Steuersignale bildet, mit denen die weitere Behandlung der Behälter 7 gesteuert wird. Auf diese Weise ist es möglich, die Behälter in Abhängigkeit von den Mängeln, mit denen sie behaftet sind, weiter zu behandeln. Behälter, in denen durch Vergleich der Spektren gesundheitsschädliche Substanzen entdeckt werden, können von jeder Weiterverarbeitung ausgeschlossen werden. Zeigt der Vergleich der Spektren, daß die Behälterinnenwand Reste von Aromastoffen aus früheren Füllungen enthält, so können diese Behälter zu Füllstationen transportiert werden, wo die gleiche Art von Flüssigkeit wieder eingefüllt wird. Fremdbehälter, also Behälter aus einem anderen Kunststoff oder Glasmaterial, können entsprechend sortiert und weiterbehandelt werden.

In einer Lernphase des Prüfsystems können die jeweils ermittelten Spektren analysiert und ihre charakteristischen Spektralliniendaten in der Speichereinheit 32 abgelegt werden, um später für den Vergleich mit den aktuellen Prüfdaten benutzt werden zu können.

Eine weitere Komponente der Streustrahlung an der Behälterinnenwand 17 geht auf eine Fluoreszenzstrahlung zurück, die durch die einfallende Prüfstrahlung in verschiedenen Substanzen erzeugt wird. Diese Fluoreszenzstrahlung wird wie die Raman-Strahlung am Beugungsgitter 27 gebeugt und dadurch spektral zerlegt. Das entstehende Spektrum wird auf dem CCD-Sensor 28 oder einem Diodenzeilen-Detektor abgebildet. Hierbei handelt es sich um ein stärker in den langwelligen Bereich des elektromagnetischen Spektrums verschobenes, lichtstarkes kontinuierliches Spektrum, dessen charakteristische Daten wieder an den Prozessor 31 übermittelt und von diesem, wie oben beschrieben, weiterverarbeitet werden. Die Spektralanalyse der Fluoreszenz-Strahlung gibt Aufschluß über Fremdstoffe wie Öle, Treibstoffe, bestimmte Lösungsmittel und andere. Solche Stoffe werden auch in sehr geringen Konzentrationen bis hinab in den ppb-Bereich (ppb = parts per billion) erkannt. Die Auswertung der Fluoreszenzstrahlung kann mit einem zweiten Gerät erfolgen, das ähnlich aufgebaut sein kann wie das im Zusammenhang mit der Auswertung der Raman-Strahlung beschriebene. In manchen Fällen kann es auch genügen, nur die Fluoreszenzstrahlung auszuwerten.

Die von der Prüfstrahlung 21 abgeleitete Referenzstrahlung 19 bildet im Sensor 18 ein Referenzsignal, das zum Eichen und zum Nullabgleich an die Prozessoren 31 und 33 übermittelt wird.

Um die Zuverlässigkeit der Prüfung zu erhöhen, können an jedem Behälter mehrere Messungen vorgenommen werden. Dazu ist vorgesehen, den jeweils in der Prüfzone 8 befindlichen Behälter 7 zwischen aufeinanderfolgenden Messungen um einen bestimmten Winkel zu drehen. So können unterschiedliche Abschnitte der Behälterinnenwand kontrolliert werden. Die Meßergebnisse können gemittelt werden, um für jeden Behälter ein einziges Prüfergebnis zu erhalten.

Das beschriebene Verfahren entdeckt also mit hoher Zuverlässigkeit auch geringe Mengen von gesundheitsschädlichen und unerwünschten Fremdstoffen in für die Wiederbefüllung zurückgeführten Mehrweg-Behältern und erhöht so die Akzeptanz der mehrfachen Verwendung dieser Getränkebehälter. Es kann mit anderen Meß- und Prüfverfahren kombiniert werden, mit denen z.B. Fehler wie Beschädigungen, größere Fremdkörper im Innern, Verformungen und dergl. entdeckt werden können.

Für die Auswertung der Raman- und Fluoreszenzstrahlung ist oben eine spezielle, derzeit bevorzugte Spektrometeranordnung beschrieben worden. Natürlich können auch andere Spektrometer eingesetzt werden wie beispielsweise Prismenspektrometer, Interferometer oder solche mit Verlaufsinterferenzfilter. Auch der Strahlengang kann abgewandelt werden, solange nur die spektrale Untersuchung der empfangenen Raman- und Fluoreszenzstrahlung gewährleistet bleibt.

## Patentansprüche

1. Verfahren zum optischen Prüfen transparenter Behälter, insbesondere von farblosen Kunststoffflaschen, wobei:
eine optische Prüfstrahlung auf eine zu prüfende Behälterinnenwand ausgerichtet wird,
die infolge des Einfalls der Prüfstrahlung von der beleuchteten Behälterflasche ausgehende durch im Bereich der Behälterinnenwand vorhandene Substanz veränderte Streustrahlung, eingeschlossen die Ramanstrahlung, erfaßt wird;
diese Streustrahlung zur Bildung der Prüfsignale analysiert und ausgewertet wird,
diese Daten des bei der Prüfung erzeugten Spektrums mit den Daten von gespeicherten
Spektren verglichen werden und
Vergleichssignale erhalten werden, die den geprüften Behälter klassifizieren.

2. Verfahren nach Anspruch 1, **dadurch gekennzeichnet, daß** die Prüfstrahlung auf einen Abschnitt der Behälterinnenwand fokussiert wird und daß eine in diesem Abschnitt durch den Einfall der Prüfstrahlung erzeugte Streustrahlung erfaßt und zur Bildung von wenigstens eine charakteristische Eigenschaft der Innenwandfläche repräsentierenden Prüfsignalen analysiert und verarbeitet wird.

3. Verfahren nach Anspruch 1 oder 2. **dadurch gekennzeichnet, daß**
- durch die einfallende Prüfstrahlung wenigstens eine im Bereich der Behälterinnenwand vorhandene Substanz zur Abgabe einer Fluoreszenzstrahlung angeregt wird,
- die Fluoreszenzstrahlung erfaßt und spektral zerlegt wird und
- in Abhängigkeit von der Struktur des Fluoreszenzspektrums charakteristische Qualitätsmerkmale und/oder Bestandteile im Bereich der Behälterinnenwand repräsentierende Prüfsignale gebildet werden.

4. Verfahren nach Anspruch 1 bis 3, **dadurch gekennzeichnet, daß** die Streu- und/oder Fluoreszenzstrahlung zur Bildung eines Beugungsspektrums an einem Beugungsgitter spektral zerlegt wird.

5. Verfahren nach einem der Ansprüche 1 bis 4, **dadurch gekennzeichnet, daß**
- charakteristische Daten wenigstens eines Streulicht- und/oder Fluoreszenzlichtspektrums gespeichert werden, daß entsprechende charakteristische Daten jedes beim Prüfen eines Behälters aktuell erzeugten Spektrums mit den gespeicherten Daten verglichen und entsprechende Vergleichssignale gebildet werden und daß die geprüften Behälter in Abhängigkeit von den Vergleichssignalen als ordnungsgemäß,, á oder fehlerhaft klassifiziert werden.

6. Verfahren nach einem der Ansprüche 1 bis 5, **dadurch gekennzeichnet, daß** die charakteristischen Daten der Streulicht und/oder Fluoreszenzlichtspektren ausgewählter Substanzen gespeichert werden, daß entsprechende charakteristische Daten jedes beim Prüfen eines Behälters aktuell erzeugten Spektrums mit den gespeicherten Daten der ausgewählten Substanzen verglichen und entsprechende Vergleichssignale gebildet werden und daß die geprüften Behälter in Abhängigkeit von den Vergleichssignalen klassifiziert werden.

7. Verfahren nach einem der Ansprüche 1 bis 6, **dadurch gekennzeichnet, daß** eine in Phase und/oder Wellenlänge gegenüber der einfallenden Prüfstrahlung unveränderte zweite Streustrahlung (Rayleigh-Streuung) erfaßt wird und daß in Abhängigkeit von der Intensität dieser zweiten Streustrahlung charakteristische Eigenschaften und/oder Bestandteile der Behälterinnenwand repräsentierende zweite Prüfsignale gebildet werden.

8. Verfahren nach einem der Ansprüche 1 bis 7, **dadurch gekennzeichnet, daß** die zu prüfenden Behälter nacheinander in den Fokusbereich der optischen Prüfstrahlung gefördert werden, daß jeder Behälter während seines Aufenthalts im Fokusbereich der Prüfstrahlung zur Bildung von wenigstens zwei Prüfsignalen wenigstens zweimal geprüft wird, daß die Position des Behälters zur einfallenden Prüfstrahlung zwischen aufeinanderfolgenden Prüfvorgängen verändert wird und daß aus den einem Behälter zugeordneten Prüfsignalen ein Mittelwertsignal erzeugt wird.

9. Verfahren nach einem der Ansprüche 1 bis 8, **dadurch gekennzeichnet, daß** als Prüfstrahlung eine Laserstrahlung im optischen Wellenlängenbereich des Spektrums verwendet wird.

10. Vorrichtung zur Durchführung des Verfahrens nach einem der vorangehenden Ansprüche zum optischen Prüfen transparenter Behälter, insbesondere von farblosen Kunststoffflaschen, mit:
- einem die zu prüfenden Behälter nacheinander in eine Prüfzone fördernden Behälterförderer,
- einer Prüfeinrichtung, die eine Strahlungsquelle zum Aussenden einer optischen Prüfstrahlung, Strahlführungsmittel zum Führen und Ausrichten der Prüfstrahlung auf einen zu prüfenden Behälter in der Prüfzone und zum Empfangen und Führen einer aufgrund des Einfalls der Prüfstrahlung von der Prüfzone ausgehenden Meßstrahlung und Sensormittel zum Erfassen und Verarbeiten der Meßstrahlung zu entsprechenden Meßsignalen aufweist, und
- einer Auswertanordnung zum Verarbeiten der Meßsignale mit einem Prozessor (31), der die Daten der charakteristischen Linien des jeweils bei einer Prüfung aktuell erzeugten Linienspektrums mit den Daten der gespeicherten Spektren vergleicht und Vergleichssignale bildet, welche die geprüften Behälter entsprechend klassifizieren, wobei die Strahlungsquelle (12) zum Erzeugen einer im Bereich des Behälterinneren eine Streustrahlung hervorrufenden Prüfstrahlung (21) ausgebildet ist und die Prüfeinrichtung (2) Mittel (16, 29, 28) zum Erfassen und Analysieren dieser Streustrahlung und zur Bildung entsprechender Prüfsignale aufweist.

11. Vorrichtung nach Anspruch 11, **dadurch gekennzeichnet, daß** die Prüfeinrichtung (2) eine eine monochromatische Strahlung (21) erzeugende und aussendende Strahlungsquelle (12) aufweist.

12. Vorrichtung nach Anspruch 10 oder 11, **dadurch gekennzeichnet, daß** als Strahlungs-quelle (12) ein Laser vorgesehen ist, der eine Prüfstrahlung (21) im optischen Wellenlängenbereich des Spektrums abgibt.

13. Vorrichtung nach einem der Ansprüche 10 bis 12, **dadurch gekennzeichnet, daß** die Strahlführungsmittel (13,16) der Prüfeinrichtung (2) eine Einrichtung (16) zum Fokussieren der Prüfstrahlung (21) auf einen Abschnitt der Behälterinnenwand (17) eines in der Prüfzone (8) positionierten Behälters (7) aufweisen.

14. Vorrichtung nach einem der Ansprüche 10 bis 13, **dadurch gekennzeichnet, daß** die Prüfeinrichtung (2) als Sensormittel zum Erfassen und Analysieren der vom beleuchteten Abschnitt der Behälterinnenwand (17) ausgehenden Strahlung ein Spektrometer (26,27,28) aufweist.

15. Vorrichtung nach Anspruch 14, **dadurch gekennzeichnet, daß** das Spektrometer zum spektralen Zerlegen der erfaßten Strahlung ein holographisches Gitter (27) und zum Empfangen und Abtasten eines entstehenden Beugungsspektrums einen CCD Detektor (28) aufweist.

16. Vorrichtung nach einem der Ansprüche 10 bis 15, **dadurch gekennzeichnet, daß** die Auswertanordnung (3) derart ausgebildet ist, daß sie Mittel (31) zum Auswerten der Struktur des Spektrums der erfaßten Strahlung und zum Bilden entsprechender, charakteristische Qualitätsmerkmale und/oder Bestandteile im Bereich der Behälterinnenwand (17) repräsentierender Prüfsignale aufweist.

17. Vorrichtung nach einem der Ansprüche 10 bis 16, **dadurch gekennzeichnet, daß** die Auswertanordnung (3) Speichermittel (32) zum Speichern charakteristischer Daten wenigstens eines Spektrums wenigstens einer bekannten Meßstrahlung aufweist und daß eine Prozessoreinheit (31) zum Vergleichen der entsprechenden charakteristischen Daten eines aktuell erzeugten Spektrums einer beim Prüfen eines Behälters (7) erfaßten Meßstrahlung mit den gespeicherten Daten und zum Erzeugen von entsprechenden Vergleichssignalen vorgesehen ist, die die Behälter (7) in Abhängigkeit von den Vergleichssignalen als ordnungsgemäß oder fehlerhaft klassifiziert.

18. Vorrichtung nach einem der Ansprüche 11 bis 17, **dadurch gekennzeichnet, daß** die Auswertanordnung (3) Speichermittel (32) zum Speichern charakteristischer Daten wenigstens eines Spektrums wenigstens einer bekannten Meßstrahlung aufweist und daß eine Prozessoreinheit (31) zum Vergleichen der entsprechenden charakteristischen Daten eines aktuell erzeugten Spektrums einer beim Prüfen eines Behälters (7) erfaßten Meßstrahlung mit den gespeicherten Daten und zum Erzeugen von entsprechenden Vergleichssignalen vorgesehen ist, welche die Behälter (7) in Abhängigkeit von den Vergleichssignalen als ordnungsgemäß oder fehlerhaft klassifiziert.

## Claims

1. Method for optically testing transparent containers, in particular colourless plastic bottles, in which:
an optical testing radiation is aligned with a container interior to be tested,
the scattered radiation, including the Raman radiation, emanating from the illuminated container bottle as a consequence of the incidence of the testing radiation and varied by substance present in the region of the container inner wall is detected,
this scattered radiation is analysed and evaluated to form the test signals,
these data of the spectrum generated during testing are compared with the data of stored spectra, and
reference signals are obtained which classify the tested container.

2. Method according to Claim 1, **characterized in that** the testing radiation is focussed onto a section of the container inner wall, and **in that** a scattered radiation generated in this section by the incidence of the testing radiation is detected and analysed and processed to form test signals representing at least one characteristic property of the inner wall surface.

3. Method according to Claim 1 or 2, **characterized in that**
- at least one substance present in the region of the container inner wall is excited to output a fluorescence radiation by the incident testing radiation,
- the fluorescence radiation is detected and spectrally decomposed, and
- test signals representing characteristic quality features and/or constituents in the region of the container inner wall are formed as a function of the structure of the fluorescence spectrum.

4. Method according to Claims 1 to 3, **characterized in that** the scattered and/or fluorescence radiation is spectrally decomposed on a diffraction grating to form a diffraction spectrum.

5. Method according to one of Claims 1 to 4, **characterized in that**
- characteristic data of at least one scattered light and/or fluorescent light spectrum are stored, **in that** corresponding characteristic data of each spectrum currently generated during testing of a container are compared with the stored data and corresponding reference signals are formed, and **in that** the tested containers are classified as in order or defective as a function of the reference signals.

6. Method according to one of Claims 1 to 5, **characterized in that** the characteristic data of the scattered light and/or fluorescence light spectra of selected substances are stored, **in that** corresponding characteristic data of each spectrum currently generated during testing of a container are compared with the stored data of the selected substances, and corresponding reference signals are formed, and **in that** the tested containers are classified as a function of the reference signals.

7. Method according to one of Claims 1 to 6, **characterized in that** a second scattered radiation (Rayleigh scattering) unchanged in phase and/or wavelength by comparison with the incident testing radiation is detected, and **in that** second test signals representing characteristic properties and/or constituents of the container inner wall are formed as a function of the intensity of this second scattered radiation.

8. Method according to one of Claims 1 to 7, **characterized in that** the containers to be tested are conveyed sequentially into the focal region of the optical testing radiation, **in that** each container is tested at least twice during its stay in the focal region of the testing radiation in order to form at least two test signals, **in that** the position of the container in relation to the incident testing radiation is varied between consecutive test operations, and **in that** an average signal is generated from the test signals assigned to a container.

9. Method according to one of Claims 1 to 8, **characterized in that** a laser radiation in the optical wavelength region of the spectrum is used as testing radiation.

10. Apparatus for carrying out the method according to one of the preceding claims for optically testing transparent containers, in particular colourless plastic bottles, having,
- a container conveyor sequentially conveying the containers to be tested into a testing zone,
- a testing device which has a radiation source for emitting an optical testing radiation, beam-guiding means for guiding and aligning the testing radiation with a container to be tested in the testing zone, and for receiving and guiding a measuring radiation emanating from the testing zone because of the incidence of the testing radiation, and sensor means for detecting and processing the measuring radiation to form corresponding measuring signals, and
- an evaluation arrangement for processing the measuring signals with the aid of a processor (31), which compares the data of the characteristic lines of the line spectrum, currently generated in each case during testing, with the data of the stored spectra, and forms reference signals which classify the tested containers correspondingly, the radiation source (12) being designed for generating a reference radiation (21) causing a scattered radiation in the region of the container interior, and the testing device (2) having means (16, 29, 28) for detecting and analysing this scattered radiation and for forming corresponding test signals.

11. Apparatus according to Claim 11, **characterized in that** the testing device (2) has a radiation source (12) generating and emitting a monochromatic radiation (21).

12. Apparatus according to Claim 10 or 11, **characterized in that** provided as radiation source (12) is a laser which outputs a test radiation (21) in the optical wavelength region of the spectrum.

13. Apparatus according to one of Claims 10 to 12, **characterized in that** the beam-guiding means (13, 16) of the testing device (2) have a device (16) for focussing the testing radiation (21) onto a section of the container inner wall (17) of a container (7) positioned in the testing zone (8).

14. Apparatus according to one of Claims 10 to 13, **characterized in that** the testing device (2) has a spectrometer (26, 27, 28) as sensor means for detecting and analysing the radiation emanating from the illuminated section of the container inner wall (17).

15. Apparatus according to Claim 14, **characterized in that** the spectrometer has a holographic grating (27) for spectrally decomposing the detected radiation, and a CCD detector (28) for receiving and scanning a diffraction spectrum produced.

16. Apparatus according to one of Claims 10 to 15, **characterized in that** the evaluation arrangement (3) is designed in such a way that it has means (31) for evaluating the structure of the spectrum of the detected radiation and for forming corresponding test signals representing characteristic quality features and/or constituents in the region of the container inner wall (17).

17. Apparatus according to one of Claims 10 to 16, **characterized in that** the evaluation arrangement (3) has storage means (32) for storing characteristic data of at least one spectrum of at least one known measuring radiation, and **in that** a processor unit (31) is provided for comparing the corresponding characteristic data of a currently generated spectrum of a measuring radiation, detected during testing of a container (7), with the stored data, and for generating corresponding reference signals, which unit classifies the containers (7) as in order or defective as a function of the reference signals.

18. Apparatus according to one of Claims 11 to 17, **characterized in that** the evaluation arrangement (3) has storage means (32) for storing characteristic data of at least one spectrum of at least one known measuring radiation, and **in that** a processor unit (31) is provided for comparing the corresponding characteristic data of a currently generated spectrum of a measuring radiation, detected during testing of a container (7), with the stored data, and for generating corresponding reference signals, which unit classifies the containers (7) as in order or defective as a function of the reference signals.

## Revendications

1. Procédé pour le contrôle optique de récipients transparents, notamment des bouteilles en plastique incolores, avec lequel :
un rayonnement de contrôle optique est dirigé sur une paroi intérieure du récipient à contrôler ;
le rayonnement diffusé, y compris le rayonnement Raman, modifié par la substance présente dans la zone de la paroi intérieure du récipient et sortant de la bouteille récipient éclairée du fait de l'incidence du rayonnement de contrôle est détecté ;
ce rayonnement diffusé est analysé puis évalué pour former les signaux de contrôle ;
ces données du spectre généré lors du contrôle sont comparées avec les données de spectres mémorisés et
des signaux de comparaison sont obtenus, lesquels classifient le récipient contrôlé.

2. Procédé selon la revendication 1, **caractérisé en ce que** le rayonnement de contrôle est concentré sur une section de la paroi intérieure du récipient et qu'un rayonnement diffusé provoqué dans cette section par l'incidence du rayonnement de contrôle est détecté puis analysé et traité pour former au moins une propriété caractéristique des signaux de contrôle représentant la surface de la paroi intérieure.

3. Procédé selon la revendication 1 ou 2, **caractérisé en ce que**
- au moins une substance dans la zone de la paroi intérieure du récipient est excitée par le rayonnement de contrôle incident pour délivrer un rayonnement fluorescent ;
- le rayonnement fluorescent est détecté puis décomposé en un spectre et
- des signaux de contrôle représentatifs des caractéristiques de qualité et/ou des composantes dans la zone de la paroi intérieure du récipient sont formés en fonction de la structure du spectre de fluorescence.

4. Procédé selon la revendication 1 à 3, **caractérisé en ce que** le rayonnement diffusé et/ou fluorescent est décomposé en un spectre sur un réseau de diffraction pour former un spectre de diffraction.

5. Procédé selon l'une des revendications 1 à 4, **caractérisé en ce que**
- les données caractéristiques d'au moins un spectre de lumière diffusée et/ou de lumière fluorescente sont mémorisées, que les données caractéristiques correspondantes de chaque spectre effectivement généré lors du contrôle du récipient sont comparées avec les données mémorisées et des signaux de comparaison correspondants sont formé et que les récipients contrôlés sont classifiés comme corrects ou défectueux en fonction des signaux de comparaison.

6. Procédé selon l'une des revendications 1 à 5, **caractérisé en ce que** les données caractéristiques de la lumière diffusée et/ou des spectres de lumière fluorescente de substances choisies sont mémorisées, que les données caractéristiques correspondantes de chaque spectre effectivement généré lors du contrôle d'un récipient sont comparées avec les données mémorisées des substances choisies et des signaux de comparaison correspondants sont formé et que les récipients contrôlés sont classifiés en fonction des signaux de comparaison.

7. Procédé selon l'une des revendications 1 à 6, **caractérisé en ce qu'**un deuxième rayonnement diffusé (rayonnement de Rayleigh) dont la phase et/ou la longueur d'onde est inchangée par rapport à celle du rayonnement de contrôle incident est détecté et que des deuxièmes signaux de contrôle représentant les propriétés et/ou les composantes caractéristiques. de la paroi intérieure du récipient sont formés en fonction de l'intensité de ce deuxième rayonnement diffusé.

8. Procédé selon l'une des revendications 1 à 7, **caractérisé en ce que** les récipients à contrôler sont acheminés l'un après l'autre dans la zone de concentration du rayonnement de contrôle optique, que chaque récipient est contrôlé au moins deux fois pendant son séjour dans la zone de concentration du rayonnement optique afin de former au moins deux signaux de contrôle, que la position du récipient par rapport au rayonnement de contrôle incident est modifiée entre les opérations de contrôle successives et qu'un signal de valeur moyenne est généré à partir des signaux de contrôle associés à un récipient.

9. Procédé selon l'une des revendications 1 à 8, **caractérisé en ce que** le rayonnement de contrôle utilisé est un rayonnement laser dans la plage de longueur d'onde optique du spectre.

10. Appareillage pour réaliser le procédé selon l'une des revendications précédentes pour le contrôle optique de récipients transparents, notamment des bouteilles en plastique incolores, avec :
- un convoyeur de récipient qui transporte les récipients à contrôler les uns après les autres dans une zone de contrôle,
- un dispositif de contrôle qui présente une source de rayonnement pour émettre un rayonnement de contrôle optique, des moyens de guidage du rayonnement pour guider et aligner le rayonnement de contrôle sur un récipient à contrôler dans la zone de contrôle et pour recevoir et guider un rayonnement de mesure sortant de la zone de contrôle du fait de l'incidence du rayonnement de contrôle et des moyens de détection pour détecter et traiter le rayonnement de mesure en des signaux de mesure correspondant, et
- un arrangement d'évaluation pour traiter les signaux de mesure avec un processeur (31), lequel compare les données des courbes caractéristiques du spectre de raies effectivement généré lors d'un contrôle avec les données des spectres mémorisés et forme des signaux de comparaison qui classifient en conséquence les récipients contrôlés, la source de rayonnement (12) étant configurée de manière à générer un rayonnement de contrôle (21) provoquant un rayonnement diffusé dans la zone de l'intérieur du récipient et le dispositif de contrôle (2) présentant des moyens (16, 29, 28) pour détecter et analyser ce rayonnement diffusé et pour former des signaux de contrôle correspondants.

11. Appareillage selon la revendication 10, **caractérisé en ce que** le dispositif de contrôle (2) présente une source de rayonnement (12) produisant et émettant un rayonnement monochromatique (21).

12. Appareillage selon la revendication 10 ou 11, **caractérisé en ce que** la source de rayonnement (12) prévue est un laser qui délivre un rayonnement de contrôle (21) dans la plage de longueur d'onde optique du spectre.

13. Appareillage selon l'une des revendications 10 à 12, **caractérisé en ce que** les moyens de guidage du rayonnement (13, 16) du dispositif de contrôle (2) présentent un dispositif (16) pour concentrer le rayonnement de contrôle (21) sur une section de la paroi intérieure du récipient (17) d'un récipient (7) positionné dans la zone de contrôle (8).

14. Appareillage selon l'une des revendications 10 à 13, **caractérisé. en ce que** le dispositif de contrôle (2) présente un spectromètre (26, 27, 28) comme moyen de détection pour détecter et analyser le rayonnement émis par la section éclairée de la paroi intérieure du récipient (17).

15. Appareillage selon la revendication 14, **caractérisé en ce que** le spectromètre présente une grille holographique (27) pour la décomposition spectrale du rayonnement reçu et un capteur CCD (28) pour recevoir et échantillonner un spectre de diffraction produit.

16. Appareillage selon l'une des revendications 10 à 15, **caractérisé en ce que** l'arrangement d'évaluation (3) est configuré de telle manière qu'il présente des moyens (31) pour évaluer la structure du spectre du rayonnement détecté et pour former des signaux de contrôle représentant des caractéristiques de qualité et/ou des composantes caractéristiques correspondantes dans la zone de la paroi intérieure du récipient (17).

17. Appareillage selon l'une des revendications 10 à 16, **caractérisé en ce que** l'arrangement d'évaluation (3) présente des moyens de mémorisation (32) pour mémoriser les données caractéristiques d'un spectre d'au moins un rayonnement de mesure connu et qu'une unité à processeur (31) est prévue pour comparer les données caractéristiques correspondantes d'un spectre effectivement généré d'un rayonnement de mesure détecté lors du contrôle d'un récipient (7) avec les données mémorisées et pour produire des signaux de comparaison correspondants qui classifient le récipient (7) en tant que correct ou défectueux en fonction des signaux de comparaison.

18. Appareillage selon l'une des revendications 11 à 17, **caractérisé en ce que** l'arrangement d'évaluation (3) présente des moyens de mémorisation (32) pour mémoriser les données caractéristiques d'un spectre d'au moins un rayonnement de mesure connu et qu'une unité à processeur (31) est prévue pour comparer les données caractéristiques correspondantes d'un spectre effectivement généré d'un rayonnement de mesure détecté lors du contrôle d'un récipient (7) avec les données mémorisées et pour produire des signaux de comparaison correspondants qui classifient le récipient (7) en tant que correct ou défectueux en fonction des signaux de comparaison.
